# EUROPEAN PATENT APPLICATION

(11) **EP 3 546 445 A1**
(43) Date of publication of application: **02.10.2019**
(21) Application number: 19163730.5
(22) Date of filing: 19.03.2019
(51) Int. Cl.: C07C 209/86, C07C 211/36

(54) **PROCESS TO INCREASE THE FRACTION OF TRANS-ISOMERS OF A DI-AMINO-COMPOUND**

(30) Priority: 27.03.2018 EP 18164234
(71) Applicant: BASF SE, 67056 Ludwigshafen am Rhein (DE)
(72) Inventor: WLOCH, Sebastian, 67056 Ludwigshafen (DE); SCHULZ, Lukas, 67056 Ludwigshafen (DE); PANCHENKO, Alexander, 67056 Ludwigshafen (DE); WEIDERT, Jan-Oliver, 67056 Ludwigshafen (DE)
(74) Representative: BASF IP Association

(57) **Abstract**

A process to increase the fraction of trans-isomers of a di-amino-compound selected from 2,4-diamino-1-methylcyclohexane, 2,6-diamino-1-methylcyclohexane or mixtures thereof, and comprising trans-isomers having the two amino groups of the di-amino compound in trans position to the cyclohexane ring as well as cis-isomers having the two amino groups in cis position to the cyclohexane ring wherein a solution comprising the di-amino-compound and an auxiliary compound with at least one hydroxy group having a molecular weight of at maximum 1000 g/mol is prepared, the temperature of the solution is decreased until a suspension comprising crystals is obtained, the crystals are separated from the suspension, and a liquid comprising a trans-enriched di-amino-compound with an increased fraction of trans-isomers is obtained.

## Description

Object of the present invention is a process to increase the fraction of trans-isomers of a di-amino-compound selected from 2,4-diamino-1-methylcyclohexane, 2,6-diamino-1-methylcyclohexane or mixtures thereof, and comprising trans-isomers having the two amino groups of the di-amino compound in trans position to the cyclohexane ring as well as cis-isomers having the two amino groups in cis position to the cyclohexane ring
wherein
- a solution comprising the di-amino-compound and an auxiliary compound with at least one hydroxy group having a molecular weight of at maximum 1000 g/mol is prepared,
- the temperature of the solution is decreased until a suspension comprising crystals is obtained,
- the crystals are separated from the suspension,
- and a liquid comprising a trans-enriched di-amino-compound with an increased fraction of trans-isomers is obtained.

Compounds with two amino groups are used in various technical applications. They are used in chemical synthesis, for example for the synthesis of di-isocyanates. Compounds with two amino groups are also used as monomers in polymerization, for example for the preparation of poly-ureas or polyurethanes.

If the two amino groups of the compound are bonded to a six-membered ring system both amino groups may be found on the same side of the ring-system (cis-isomer) or on opposite side of the ring system (trans-isomer). Such isomerism has an impact on the physical and chemical properties and hence on the suitability of the compound for certain technical applications.

Compounds such as 2,4-diamino-1-methylcyclohexane, 2,6-diamino-1-methylcyclohexane or mixtures thereof usually are mixtures of trans-isomers and cis-isomers.

The separation of trans-isomers and cis-isomers of 2,4-diamino-1-methylcyclohexane, 2,6-diamino-1-methylcyclohexane or mixtures thereof by distillation is described in WO 2016/083210. As the boiling points of the trans-isomers and cis-isomers are rather close, the distillation results in two phases, one trans-enriched phase and one cis-enriched phase which still comprise some fraction of the other isomer. Furthermore, the distillation requires high energy.

WO 2017/093308 discloses a process for the isomerization of 2,4-diamino-1-methylcyclohexane, 2,6-diamino-1-methylcyclohexane or mixtures thereof at elevated temperatures in contact with an heterogenous catalyst.

It was an object of this invention to provide an alternative process for the separation of trans-isomers and cis-isomers of 2,4-diamino-1-methylcyclohexane, 2,6-diamino-1-methylcyclohexane or mixtures thereof. The alternative process should allow an effective separation of trans-isomers and cis-isomers. Furthermore, the alternative process should be easy to perform and should consume as low energy as possible.

Accordingly, the process defined above has been found.

### To the di-amino-compound

The di-amino-compound is selected from 2,4-diamino-1-methylcyclohexane, 2,6-diamino-1-methylcyclohexane or mixtures thereof.

Such di-amino-compounds are notably obtained by hydrogenation of 2,4 - and/or 2,6- di-amino-toluene. Usually a mixture of 2,4 - and/or 2,6- di-amino-toluene is used for the hydrogenation thus obtaining a mixture of 2,4-diamino-1-methylcyclohexane and 2,6-diamino-1-methylcyclohexane.

Preferably, the di-amino-compound is a mixture of 2,4-diamino-1-methylcyclohexane and 2,6-diamino-1-methylcyclohexane. Most preferably, the diamino-compound comprises 50 to 95 % by weight of 2,4-diamino-1-methylcyclohexane and 5 to 50 % by weight of 2,6-diamino-1-methylcyclohexane.

The mixture may also comprise other compounds with two amino groups, such as, for example, 2,3-diaminomethylcyclohexane and 3,4-diaminomethylcyclohexane. The proportion of other compounds with two amino groups is typically 0% to 5% by weight, notably 0 to 1 by weight, most preferably 0 to 0.5, respectively o to 0.1% by weight, based on the total weight of the di-amino-compound.

The di-amino-compound may comprise the following cis- and trans isomers:

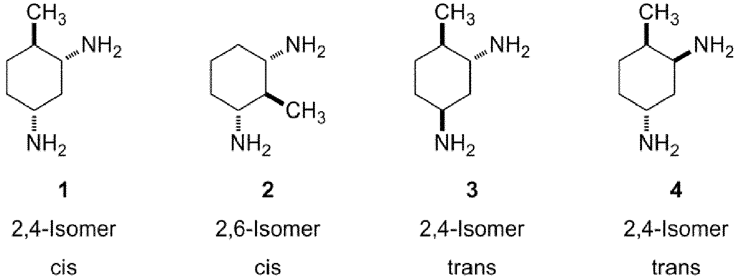

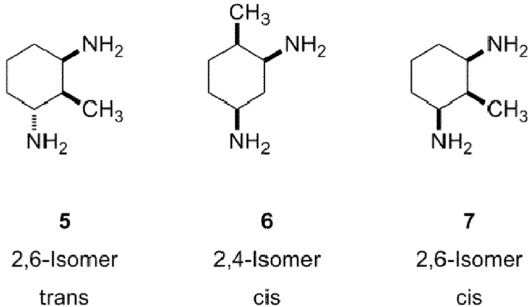

Compounds 2 and 7 are meso forms and do not have enantiomers. Compounds 1 and 3 - 6 have tow enantiomeric forms (mirror and mirror image). Any reference to compounds 1 and 3-6 includes both enantiomeric forms of compounds 1 and 3-6.

In the process of this invention a di-amino-compound as originally obtained from hydrogenation may be used. Alternatively, a di-amino compound which has already undergone further work-up, for example in processes which alter the original composition and the original portion of cis- and trans isomers may be used. Such a process is for example the distillation process disclosed in WO 2016/083210.

In a preferred embodiment, the di-amino-compound comprises
0 to 80 % by weight of the 2,4-isomer cis (1)
0.1 to 80 % by weight of the 2,6-isomer cis (2)
0.1 to 80 % by weight of the 2,4-isomer trans (3)
0.1 to 80 % by weight of the 2,4-isomer trans (4)
0.1 to 80 % by weight of the 2,6-isomer trans (5)
0 to 80 % by weight of the 2,4-isomer cis (6)
0 to 80 % by weight of the 2,6-isomer cis (7) and
0 to 5 % by weight of other compounds with two amino groups
based on the total amount of the di-amino compound.

In a more preferred embodiment, the di-amino-compound comprises
0 to 60 % by weight of the 2,4-isomer cis (1)
0.1 to 30 % by weight of the 2,6-isomer cis (2)
0.1 to 50 % by weight of the 2,4-isomer trans (3)
0.1 to 40 % by weight of the 2,4-isomer trans (4)
0.1 to 30 % by weight of the 2,6-isomer trans (5)
0 to 20 % by weight of the 2,4-isomer cis(6)
0 to 10 % by weight of the 2,6-isomer cis (7)
0 to 5 % by weight of other compounds with two amino groups
based on the total amount of the di-amino compound.

In a most preferred embodiment, the di-amino-compound comprises
0 to 10 % by weight of the 2,4-isomer cis (1)
5 to 30 % by weight of the 2,6-isomer cis (2)
10 to 45 % by weight of the 2,4-isomer trans (3)
10 to 35 % by weight of the 2,4-isomer trans (4)
5 to 25 % by weight of the 2,6-isomer trans (5)
0 to 5 % by weight of the 2,4-isomer cis (6)
0 to 5 % by weight of the 2,6-isomer cis (7)
0 to 5 % by weight of other compounds with two amino groups
based on the total amount of the di-amino compound. Such a composition is, for example, available as product of the above-mentioned distillation process.

In a preferred embodiment, the total amount of trans-isomers of the di-amino-compound is 20 to 95 % by weight, notably 70 to 95 % by weight and the total amount of cis-isomers of the di-amino-compound is preferably 5 to 80% by weight, notably 5 to 30 % by weight.

### To the auxiliary compound

The auxiliary compound has at least one hydroxy group and has a molecular weight of at maximum 1000 g/mol.

Preferably, the molecular weight of the auxiliary compound is at maximum 500 g/mol and more preferably at maximum 200 g/mol. Preferably, the molecular weight of the auxiliary compound is at minimum 46, more preferably at minimum 62 g/mol.

Preferably, the auxiliary compound comprises at maximum five, notably at maximum four hydroxy groups.

More preferably, the auxiliary compound comprises two or three hydroxy groups.

In a most preferred embodiment, the auxiliary compound comprises two hydroxy groups.

Preferably, the auxiliary does not comprise any other atoms than hydrogen, carbon and oxygen. The auxiliary compound may comprise oxygen also in other form than hydroxy groups, for example in form of ether groups.

In a particularly preferred embodiment, the auxiliary compound is an aliphatic compound which comprises at least one hydroxy group and which may furthermore comprise ether groups.

In a more preferred embodiment, the auxiliary compound is an (cyclo)aliphatic compound which comprise at least one hydroxy group and no ether groups or other functional groups.

Example of suitable auxiliary compounds are
alkanols, notably C2- to C8-alkanols such as ethanol, propanol, butanol, pentanol
alkane-diols, notably C2 to C8- alkane-diols, such as ethane-diol, propane-diol, specifically propane-1,3-diol, butane-diol, specifically butane-1,4-diol,
cyclohexane substituted by at least one hydroxy groups or substituted by at least one hydroxyalkyl group, notably cyclohexane-1,2-diol, cyclohexane-1,3-diol and cyclohexane-1,2-dimethanol.
compounds having three or four hydroxy groups are, for example, glycerol, trimethylolpropane, hexane-1,3,6-triol, hexane-1,2,6-triol, diglycerol and pentaerythritol.

Most preferably, the auxiliary compound is an alkane-diol with 2 to 8 carbon atoms.

In particular, the auxiliary compound is butane-1,4-diol.

### To the process

First, - a solution comprising the di-amino-compound and the auxiliary compound is prepared.

The di-amino-compound has a melting point significantly below 0°C. Mixtures of 2,4-diamino-1-methylcyclohexane and of 2,6-diamino-1-methylcyclohexane have a melting point below -60°C, probably even much lower.

By adding the auxiliary compound, the melting point increases.

Preferably, the obtained mixture of auxiliary compound and di-amino-compound has a melting point of -20 to +120°C, more preferably of 0 to +100°C and most preferably from +20 to +50°C. At temperatures higher than the melting point, the auxiliary compound and the di-amino-compound form a solution.

Preferably, the solution comprises 5 to 60% by weight of the auxiliary compound and 40 to 95% by weight of the di-amino-compound. More preferably, the solution comprises 10 to 50% by weight of the auxiliary compound and 50 to 90% by weight of the di-amino-compound. Most preferably, the solution comprises 15 to 45% by weight of the auxiliary compound and 55 to 85% by weight of the di-amino-compound.

In the next process step, the temperature of the solution is decreased by cooling until a suspension comprising crystals is obtained, hereinafter referred to as crystallization process. The crystallization process may be performed in crystallizers that are commercially available.

The crystallization process may be supported by first preparing a seed of crystals. Different methods of seeding are known and may be used. In a preferred embodiment, the seed is prepared by cooling the solution below the melting point until first crystals are seen and then increasing the temperature again to a temperature slightly below the melting point. Thus, a solution comprising small amounts of a seed is obtained.

In the crystallization process, the temperature of the solution is decreased starting preferably from a temperature of -20 to +120 °C, more preferably from a temperature of 0 to +100°C and most preferably from a temperature of +20 to +50°C and terminating the cooling preferably at a temperature -35 to +105°C, more preferably at -15 to +85°C and most preferably at 0 to +35°C. In a preferred embodiment, the cooling is started at a temperature above the melting point or at the melting point. Cooling is terminated when sufficient crystals have been formed. Usually the final temperature is from 0 to +35°C, notably from +5 to +20°C.

The process may be performed in a batch process or continuously.

In a batch process, the temperature of the solution is preferably decreased in a rate of -0.5 to - 20 Kelvin per hour, notably of -1 to 10 Kelvin per hour.

In a continuous process, the solid-free solution may be continuously transferred from a first container (buffer tank) to a crystallizer or several crystallizers which are operated in line, whereby the buffer tank is kept at the starting temperature and the crystallizer is kept at the final temperature or -in case of several crystallizers- the crystallizers are kept at decreasing temperatures with the last one being kept at the final temperature as defined above.

The crystals are separated from the suspension in a solid-liquid separation step, for example on a centrifuge.

The remaining liquid comprises a trans-enriched di-amino-compound with an increased fraction of trans-isomers and the auxiliary compound.

Preferably, the trans-enriched di-amino-compound comprises 70% to 100% by weight of trans-isomers.

Preferably, the trans-enriched di-amino compound comprises 0 to 5 % by weight of the 2,6-isomer cis (2).

The auxiliary compound may be removed from the liquid obtained, for example by distillation.

### Examples

The di-amino-compound used was a mixture of 2,4-diamino-1-methylcyclohexane and of 2,6-diamino-1-methylcyclohexane. Such mixtures are shortly referred to as MCDA in the examples.

The specific composition of MCDA is listed for each example in a table. In the table the content of cis-isomers and trans-isomers of formulas 1 to 7 (see above formulas in the specification) is defined in weight % based on the total amount of MCDA. The content of the isomers 1 to 7 was determined by gas chromatography.

In the examples, butane-1,4-diol, shortly referred to as BDO, was used as solvent.

The melting point of the mixture of MCDA and BDO was determined as follows:
MCDA and BDO were mixed at an elevated temperature to obtain a clear solution. The melting point of the mixture was determined by cooling the mixture until first crystals appeared, afterwards the suspension was heated up again, until all crystals dissolved. The corresponding temperature is referred to as melting point of the solution. The melting point has been determined with a turbidity probe. A solution without any turbidity corresponds to a solution without any crystals respectively solids.

A cooling crystallization was performed as follows:
In a first step a seed of crystals has been prepared using a so called "seeding loop". For the seeding loop, the solution is cooled down until first solid particles appear and afterwards the suspension is heated up again close to the melting point to dissolve some of the solid particles. A solution comprising a small number of solid particles (seed) is obtained, which is then used for the crystallization process.

The examples were performed in a one-liter double jacketed crystallizer, equipped with three baffles and a two-stage pitch blade stirrer, which was operated at 600 rpm. Furthermore, the crystallizer was equipped with a temperature and pressure sensor, as well as a turbidity probe to detect the formation of solid particles. It was possible to take samples of mother liquid which was free of solids via a filter fritte from the crystallizer, additionally a filter nutsche was mounted below the crystallizer to separate the mother liquid from the crystals.

The crystallizer was charged with 400 g of the MCDA/BDO solution.
A cooling crystallization was performed, by applying a linear cooling ramp with -1 K/h. Specific temperatures (listed in the tables) were kept constant for some hours, so that the solid-liquid equilibrium could be fully established. At these temperatures samples (about 3 g) from the solid-free mother liquid were taken and analyzed by gas chromatography.

The yield of trans-enriched mother liquid was determined in a second run. At a pre-determined temperature which usually corresponded to a temperature listed in the tables of the examples, the whole suspension was transferred from the crystallizer on the filter nutsche. The crystals were separated from the mother liquor. Both fractions crystals and mother liquor were analyzed by GC and the masses of both fractions were determined.

### Example 1

MCDA with a reduced content of cis 1, 6 und 7 has been used. The isomer composition of the MCDA used, is the composition at the starting temperature which is 40°C, see first line of the Table below.

MCDA and BDO were mixed in a ratio of 70 : 30 parts by weight. The melting point of the mixture of MDCA and BDO was +40°C.

In the Table below the weight fraction of the isomers of MCDA is listed with decreasing temperature, for each line in the table the sum of all isomers of MCDA is 100%. The composition of the MCDA in the liquid phase was measured, only.

| °C | (1) % | (2) % | (3) % | (4) % | (5) % | (6) % | (7) % | Total cis % | Total Trans % |
|---|---|---|---|---|---|---|---|---|---|
| 40 | 1.3 | 15.1 | 36.4 | 28.6 | 18.5 | 0.0 | 0.1 | 16.5 | 83.5 |
| 35.2 | 1.3 | 10.3 | 38.9 | 30.0 | 19.3 | 0.0 | 0.1 | 11.8 | 88.2 |
| 30.1 | 1.3 | 6.6 | 40.7 | 31.2 | 20.0 | 0.0 | 0.1 | 8.1 | 91.9 |
| 25.3 | 1.3 | 4.7 | 41.2 | 32.1 | 20.6 | 0.0 | 0.1 | 6.1 | 93.9 |
| 19.6 | 1.3 | 3.1 | 41.7 | 32.8 | 21.0 | 0.0 | 0.1 | 4.5 | 95.5 |
| 14.2 | 1.3 | 2.1 | 41.9 | 33.4 | 21.2 | 0.0 | 0.1 | 3.5 | 96.5 |
| Crystalsat 19.6°C | 1.0 | 45.9 | 21.4 | 19.2 | 12.4 | 0.0 | 0.1 | 46.9 | 53.1 |

The example was repeated. At 19.6°C, the crystals (comprising MCDA and BDO) were isolated from the suspension as described above. The isomer composition of the MCDA of the crystals was determined, see last line of the Table. The yield of crystals was 21.6% by weight and the yield of the liquid (also referred to as mother liquid) was 78.4% by weight (both comprising MCDA and BDO). The total yield of trans-isomers in the final mother liquid was 86% by weight of all trans-isomers of the starting solution.

### Example 2

MCDA with a reduced content of cis 1, 6 und 7 has been used. The isomer composition of the MCDA used, is the composition at the starting temperature which is 34°C, see first line of the Table below.

MCDA and BDO were mixed in a ratio of 80 : 20 parts by weight. The melting point of the mixture of MDCA and BDO was +34°C.

In the Table below the weight fraction of the isomers of MCDA is listed with decreasing temperature, for each line in the table the sum of all isomers of MCDA is 100%. The composition of the MCDA in the liquid phase was measured, only.

| °C | (1) % | (2) % | (3) % | (4) % | (5) % | (6) % | (7) % | Total cis % | Total Trans % |
|---|---|---|---|---|---|---|---|---|---|
| 34 | 1.3 | 14.8 | 36.5 | 28.7 | 18.5 | 0.0 | 0.1 | 16.2 | 83.8 |
| 20.1 | 1.3 | 6.0 | 40.8 | 31.5 | 20.2 | 0.0 | 0.1 | 7.5 | 92.5 |
| 10.5 | 1.3 | 3.6 | 41.4 | 32.7 | 20.9 | 0.0 | 0.1 | 5.0 | 95.0 |
| Crystalsat 20.1°C | 0.9 | 63.3 | 13.4 | 13.6 | 8.8 | 0.0 | 0.1 | 64.2 | 35.8 |

The example was repeated. At 20.1°C, the crystals (comprising MCDA and BDO) were isolated from the suspension as described above. The isomer composition of the MCDA of the crystals was determined, see last line of the Table. The yield of crystals was 15.5% by weight and the yield of the mother liquid was 84.5% by weight (both comprising MCDA and BDO). The total yield of trans-isomers in the final mother liquid was 93% by weight of all trans-isomers of the starting solution.

### Example 3

MCDA with a reduced content of cis 1, 6 und 7 has been used. The isomer composition of the MCDA used, is the composition at the starting temperature which is 41.5°C, see first line of the Table below.

MCDA and BDO were mixed in a ratio of 60 : 40 parts by weight. The melting point of the mixture of MDCA and BDO was +41.5°C.

In the Table below the weight fraction of the isomers of MCDA is listed with decreasing temperature, for each line in the table the sum of all isomers of MCDA is 100%. The composition of the MCDA in the liquid phase was measured, only.

| °C | (1) % | (2) % | (3) % | (4) % | (5) % | (6) % | (7) % | Total cis % | Total Trans % |
|---|---|---|---|---|---|---|---|---|---|
| 41.5 | 1.3 | 14.6 | 36.7 | 28.7 | 18.6 | 0.0 | 0.1 | 16.0 | 84.0 |
| 29.6 | 1.3 | 6.2 | 40.2 | 31.7 | 20.5 | 0.0 | 0.1 | 7.6 | 92.4 |
| 19.3 | 1.2 | 2.5 | 41.8 | 33.1 | 21.3 | 0.0 | 0.1 | 3.8 | 96.2 |
| 9.6 | 1.0 | 1.5 | 42.1 | 33.8 | 21.6 | 0.0 | 0.1 | 2.6 | 97.4 |
| Crystalsat 29.6°C | 1.0 | 45.9 | 21.4 | 19.2 | 12.4 | 0.0 | 0.1 | 46.9 | 53.1 |

The example was repeated. At 29.6°C, the crystals (comprising MCDA and BDO) were isolated from the suspension as described above. The isomer composition of the MCDA of the crystals was determined, see last line of the Table. The yield of crystals was 19.3% by weight and the yield of the mother liquid was 80.7% by weight (both comprising MCDA and BDO). The total yield of trans-isomers in the final mother liquid was 88% by weight of all trans-isomers of the starting solution.

### Example 4

MCDA with a lower cis content than in examples 1 to 3 has been used. The isomer composition of the MCDA used, is the composition at the starting temperature which is 34°C, see first line of the Table below.

MCDA and BDO were mixed in a ratio of 60 : 40 parts by weight. The melting point of the mixture of MDCA and BDO was +34°C.

In the Table below the weight fraction of the isomers of MCDA is listed with decreasing temperature, for each line in the table the sum of all isomers of MCDA is 100%. The composition of the MCDA in the liquid phase was measured, only.

| °C | (1) % | (2) % | (3) % | (4) % | (5) % | (6) % | (7) % | Total cis % | Total Trans % |
|---|---|---|---|---|---|---|---|---|---|
| 34.0 | 1.3 | 8.6 | 39.8 | 30.5 | 19.7 | 0.0 | 0.1 | 10.0 | 90.0 |
| 30.8 | 1.4 | 5.9 | 40.9 | 31.5 | 20.3 | 0.0 | 0.1 | 7.4 | 92.6 |
| 20.0 | 1.3 | 2.9 | 41.5 | 33.0 | 21.2 | 0.0 | 0.1 | 4.4 | 95.6 |
| 10.0 | 1.3 | 1.6 | 41.8 | 33.6 | 21.6 | 0.0 | 0.1 | 3.0 | 97.0 |

### Example 5

MCDA with a higher cis content than in examples 1 to 3 has been used. The isomer composition of the MCDA used, is the composition at the starting temperature which is 44.5°C, see first line of the Table below.

MCDA and BDO were mixed in a ratio of 65 : 35 parts by weight. The melting point of the mixture of MDCA and BDO was +44.5°C.

In the Table below the weight fraction of the isomers of MCDA is listed with decreasing temperature, for each line in the table the sum of all isomers of MCDA is 100%. The composition of the MCDA in the liquid phase was measured, only.

| °C | (1) % | (2) % | (3) % | (4) % | (5) % | (6) % | (7) % | Total cis % | Total Trans % |
|---|---|---|---|---|---|---|---|---|---|
| 44.5 | 1.3 | 19.7 | 34.1 | 27.2 | 17.6 | 0.0 | 0.1 | 21.1 | 78.9 |
| 40.2 | 1.3 | 5.7 | 41.1 | 31.6 | 20.2 | 0.0 | 0.1 | 7.1 | 92.9 |
| 30.2 | 1.3 | 13.6 | 37.2 | 29.0 | 18.7 | 0.0 | 0.1 | 15.1 | 84.9 |
| 20.0 | 1.3 | 2.6 | 41.9 | 33.1 | 21.0 | 0.0 | 0.1 | 4.0 | 96.0 |
| 10.4 | 1.1 | 1.9 | 41.9 | 33.6 | 21.3 | 0.0 | 0.1 | 3.1 | 96.9 |
| Crystalsat 20°C | 0.9 | 49.5 | 19.9 | 17.9 | 11.6 | 0.0 | 0.1 | 50.5 | 49.5 |

The example was repeated. At 20.0°C, the crystals (comprising MCDA and BDO) were isolated from the suspension as described above. The isomer composition of the MCDA of the crystals was determined, see last line of the Table. The yield of crystals was 35.7% by weight and the yield of the mother liquid was 64.3% by weight (both comprising MCDA and BDO). The total yield of trans-isomers in the final mother liquid was 77% by weight of all trans-isomers of the starting solution.

## Claims

1. A process to increase the fraction of trans-isomers of a di-amino-compound selected from 2,4-diamino-1-methylcyclohexane, 2,6-diamino-1-methylcyclohexane or mixtures thereof, and comprising trans-isomers having the two amino groups of the di-amino compound in trans position to the cyclohexane ring as well as cis-isomers having the two amino groups in cis position to the cyclohexane ring
wherein
- a solution comprising the di-amino-compound and an auxiliary compound with at least one hydroxy group having a molecular weight of at maximum 1000 g/mol is prepared,
- the temperature of the solution is decreased until a suspension comprising crystals is obtained,
- the crystals are separated from the suspension,
- and a liquid comprising a trans-enriched di-amino-compound with an increased fraction of trans-isomers is obtained.

2. A process according to claim 1 wherein the di-amino-compound is a mixture of 2,4-diamino-1-methylcyclohexane and 2,6-diamino-1-methylcyclohexane.

3. The process according to claim 1 or 2, wherein the di-amino-compound may comprise the following trans-isomers and cis-isomers

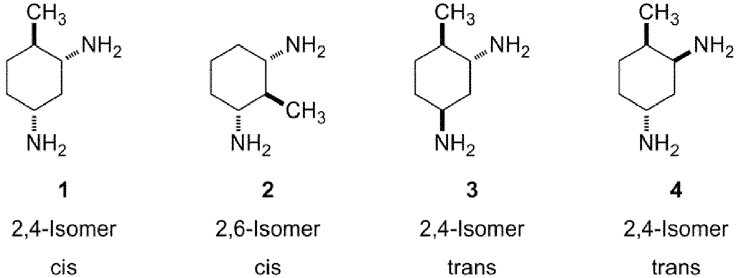

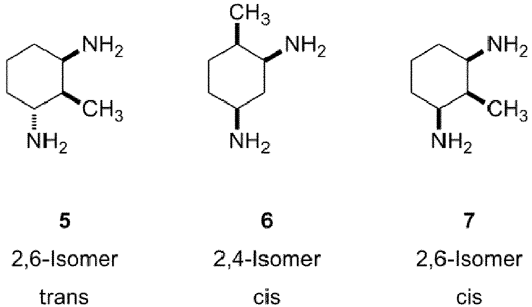

4. The process according to claim 2 and 3, wherein the di-amino-compound consists of
0 to 80 % by weight of the 2,4-isomer cis (1)
0.1 to 80 % by weight of the 2,6-isomer cis (2)
0.1 to 80 % by weight of the 2,4-isomer trans (3)
0.1 to 80 % by weight of the 2,4-isomer trans (4)
0.1 to 80 % by weight of the 2,6-isomer trans (5)
0 to 80 % by weight of the 2,4-isomer cis (6)
0 to 80 % by weight of the 2,6-isomer cis (7) and
0 to 5 % by weight of other compounds with two amino groups
based on the total amount of the di-amino compound.

5. The process according to any of claims 2 to 4, wherein the di-amino-compound consists of
0 to 10 % by weight of the 2,4-isomer cis (1)
5 to 30 % by weight of the 2,6-isomer cis (2)
10 to 45 % by weight of the 2,4-isomer trans (3)
10 to 35 % by weight of the 2,4-isomer trans (4)
5 to 25 % by weight of the 2,6-isomer trans (5)
0 to 5 % by weight of the 2,4-isomer cis (6)
0 to 5 % by weight of the 2,6-isomer cis (7)
0 to 5 % by weight of other compounds with two amino groups
based on the total amount of the di-amino compound.

6. The process according to any of claims 1 to 5, wherein 20 to 95 % by weight of the di-amino compound are trans-isomers and 5 to 80 % of the di-amino compound are cis-isomers.

7. The process according to any of claims 1 to 6, wherein the auxiliary compound is an alkane-diol with 2 to 8 carbon atoms.

8. The process according to any of claims 1 to 7, wherein the auxiliary compound is butane-1,4-diol.

9. The process according to any of claims 1 to 8, wherein the solution comprises 5 to 60 % by weight of the auxiliary compound and 40 to 95 % by weight of the di-amino-compound

10. The process according to any of claims 1 to 9, wherein the temperature of the solution is decreased from a starting temperature of +20 to +50°C to a final temperature of 0 to +35°C

11. The process according to any of claims 1 to 10, wherein the trans-enriched di-amino compound comprises 70% to 100% by weight of trans-isomers.

12. The process according to any of claims 1 to 11, wherein the trans-enriched di-amino compound comprises 0 to 5 % by weight of the 2,6-isomer cis (2).
